# EUROPÄISCHE PATENTSCHRIFT

(11) **EP 1 319 143 B1**
(45) Veröffentlichungstag und Bekanntmachung des Hinweises auf die Patenterteilung: **11.10.2006**
(21) Anmeldenummer: 01965198.3
(22) Anmeldetag: 17.08.2001
(51) Int. Cl.: F16K 7/10, A61M 39/22, A61M 5/168

(54) **VENTIL**
VALVE
SOUPAPE

(30) Priorität: 20.09.2000 DE 10046651
(43) Veröffentlichungstag der Anmeldung: 18.06.2003
(73) Patentinhaber: Fresenius Medical Care Deutschland GmbH, 61352 Bad Homburg v.d.H. (DE)
(72) Erfinder: BEDEN, Josef, 55252 Mainz-Kastel (DE); BRAUN, Jürgen, 61273 Wehrheim (DE); SCHNEIDER, Hans-Peter, 61267 Neu-Anspach (DE)
(74) Vertreter: Laufhütte, Dieter
(86) Internationale Anmeldenummer: PCT/EP2001/009510
(87) Internationale Veröffentlichungsnummer: WO 2002/025146

(56) Entgegenhaltungen:
- GB-A- 1 483 702
- GB-A- 2 331 796
- US-A- 4 333 452
- US-A- 5 178 182

## Beschreibung

Die vorliegende Erfindung betrifft ein Ventil zum Verschluß von Fluidkanälen, insbesondere zum Einsatz mit Disposablekassetten für extrakorporale Kreisläufe, wie beispielsweise der Dialyse.

Derartige Ventile werden eingesetzt, um den Fluidstrom in Dialysesystemen zuzulassen bzw. zu stoppen. Sogenannte Disposablekassetten oder Einmalkassetten umfassen in dem Kassettenkorpus Flüssigkeitskanäle, die je nach Anforderung an die Behandlung geöffnet oder verschlossen werden müssen, um die Dialyseflüssigkeit zu fördern bzw. zu unterbrechen. Die Flüssigkeitskanäle des Kassettenkorpuskönnen mit einer Disposablefolie abgeschlossen sein. Die Disposablekassette wird mit einem geräteseitigen Gegenstück verschlossen, das mit der Disposablekassette verpresst, z.B. verschraubt wird. Auf diese Weise werden die Fluidkanäle der Diposablekassette gegen die Umgebung abgeschlossen.

Die Komponenten, die gegebenenfalls mit Blut bzw. Dialyseflüssigkeit in Berührung kommen, müssen hohen Hygieneanforderungen genügen.

Bei einer in WO 94/20155 beschriebenen Disposablekassette sind in einem Korpus dieser Kassette mehrere Fluidkanäle eingeprägt. An einem Punkt, an dem der Fluidstrom unterbrochen werden können soll, sind Vorsprünge im Fluidkanal vorgesehen. Die Fluidkanäle des Korpus werden mit einem geräteseitigen Gegenstück verschlossen. Im Bereich der Vorsprünge weist dieses Gegenstück flexible Bereiche auf, die gegen die Vorsprünge in dem Fluidkanal gedrückt werden können. Auf diese Weise läßt sich der Fluidstrom unterbrechen.

Das Dokument GB 1 483 702 offenbart ein Ventil gemäß dem Oberbegriff des Anspruchs 1.

Aufgabe der vorliegenden Erfindung ist es, ein Ventil und dessen Anwendung, insbesondere zum Einsatz mit einer Disposablekassette anzugeben, die auf kostengünstige und einfache Weise den Hygieneanforderungen genügen können.

Diese Aufgabe wird mit einem Ventil mit den Merkmalen des Anspruches 1 gelöst.

Das erfindungsgemäße Ventil weist einen Ventilkörper mit einem Druckkanal auf. Eine Dichtkappe wirkt mit dem Ventilkörper derart zusammen, daß das ventilkörperseitige Ende des Druckkanales gegen die Umgebung abschließt. Zwischen dem Druckkanal und der Dichtkappe kann ein Druckraum aufgebaut werden, wobei ein verformbarer Dichtbereich der Dichtkappe in den Flüssigkeitskanal eintreten kann, um diesen zu verschließen,

Die Dichtkappe kann fest mit dem Ventilkörper verbunden sein. Besonders benutzer - bzw. wartungsfreundlich ist jedoch eine Dichtkappe, die lösbar von dem Ventilkörper ist. Dies vereinfacht und verbilligt zusätzlich die Auswechslung der hygienerelevanten Dichtkappe.

Die Ventilfunktion läßt sich einfach durch Kontrolle des Druckes in dem Druckkanal des Ventils überwachen. Durch Anlegen von Überdruck an den Druckkanal wird der Flüssigkeitskanal durch Ausdehnung des verformbaren Bereiches verschlossen. Einfache Entlüftung am Druckkanal oder Anlegen von Unterdruck öffnet das Ventil. Die verformbare Ventilkappe eröffnet einen guten Toleranzausgleich sowohl bei der Tiefe des Kanals als auch bei einem seitlichen Versatz des Ventiles gegenüber dem Flüssigkeitskanal.

Schließlich läßt sich die gesamte, einfach aufgebaute Ventileinheit leicht auswechseln. Eine Fehlfunktion des Ventiles erfordert dementsprechend nicht die Reparatur bzw. den Austausch des gesamten Gerätes bzw. geräteseitigen Gegenstückes der Disposablekassette, sondern nur des betroffenen Ventiles.

Eine bevorzugte Ausführungsform des Ventiles umfasst eine Befestigungsvorrichtung zum Befestigen des Ventilkörpers in einer Aufnahme. Mit Hilfe einer solchen Befestigungsvorrichtung läßt sich das Ventil leicht in das geräteseitige Gegenstück einer Disposablekassette einfügen. Als Befestigungsvorrichtung sind z.B. Federsprengringe oder Klammereinrichtungen denkbar. Eine einfache Ausgestaltung umfasst eine Befestigungsmutter, die auf die Außenwandung des Druckkanals auf geschraubt werden kann, der durch eine Korpuswandung des geräteseitigen Gegenstückes der Disposablekassette hindurchgesteckt wird.

Eine Dichtung ist vorgesehen, die beim Befestigen des Ventilkörpers in der Aufnahme für die Abdichtung des Druckraumes gegen die Umgebung dient. Am Ventilkörper des erfindungsgemäßen Ventils ist eine Dichtfläche vorgesehen , die von der Dichtkappe selbst derart umfasst wird, daß bei der Befestigung des Ventilkörpers die Dichtkappe an diese Dichtfläche gepresst wird. Eine gesonderte Justierung der Dichtung beim Befestigen des Ventilkörpers in der Aufnahme ist dann nicht mehr nötig. Bereits beim Anbringen der Dichtkappe an dem Ventilkörper ist auch die Dichtung bereits korrekt plaziert.

Die Dichtkappe könnte über eine separate Dichtung mit dem Flüssigkeitskanal in Verbindung gebracht werden. Die Erfindung sieht jedoch vor, daß an der Dichtkappe des erfindungsgemäßen Ventiles ein Ansatz vorgesehen ist, der gegen den Rand des Flüssigkeitskanales gedrückt werden kann, um eine entsprechende Abdichtung zu gewährleisten. Damit muß nur das Ventil bezüglich des Fluidkanales ausgerichtet sein, um eine ausreichende Abdichtung zu gewährleisten.

Bei einer vorteilhaften Weiterbildung weist die Dichtkappe einen Vorsprung auf, der den direkten Kontakt des Ventilkörpers mit dem Dichtbereich der Dichtkappe beschränkt. Durch einen solchen Vorsprung wird die Belastung der Kappe bei der Verpressung des Dichtbereiches auf den Kanal verringert. Durch die Ausbildung eines Vorsprungs wird jedoch die Bewegung des Dichtbereiches nicht beeinträchtigt.

Das erfindungsgemäße Ventil kann zur Öffnung bzw. zum Verschluß von Fluidkanälen bei verschiedenen Anwendungen eingesetzt werden. Besonders vorteilhaft läßt sich die Anordnung auf Grund ihrer Einfachheit und Verläßlichkeit jedoch zur Steuerung eines Fluidkanales in einer Disposablekassette bei der Dialyse einsetzen.

Ein erfindungsgemäßes Gerät zur Verwendung mit einer Diposablekassette weist zumindest ein erfindungsgemäßes Ventil zur Steuerung des Fluiddurchflusses in zumindest einem Fluidkanal der Disposablekassette auf. Das erfindungsgemäße Gerät zur Verwendung mit einer Disposablekassette weist auch eine Aufnahme für ein erfindungsgemäßes Ventil derart auf, dass das erfindungsgemäße Ventil zum Verschluss eines Fluidkanales der Disposablekassette eingesetzt werden kann.

Eine Disposablekassette mit mindestens einem Fluidkanal, dessen Durchfluss steuerbar sein muss, braucht dementsprechend keinerlei Disposablekassettenseitigen Aufnahmen oder Ventilsitze um mit einem erfindungsgemäßem Gerät einsetzbar zu sein.

Eine erfindungsgemäße Ausführungsform des Ventiles und seine Anwendung in einer Disposablekassette werden im folgenden anhand der anliegenden Figuren erläutert.

Dabei zeigt
- Fig. 1: den Querschnitt durch ein erfindungsgemäßes Ventil in schematischer Ansicht und
- Fig. 2: das erfindungsgemäße Ventil der Fig. 1 im Einsatz in einer, nur schematisch angedeuteten, Disposablekassette.

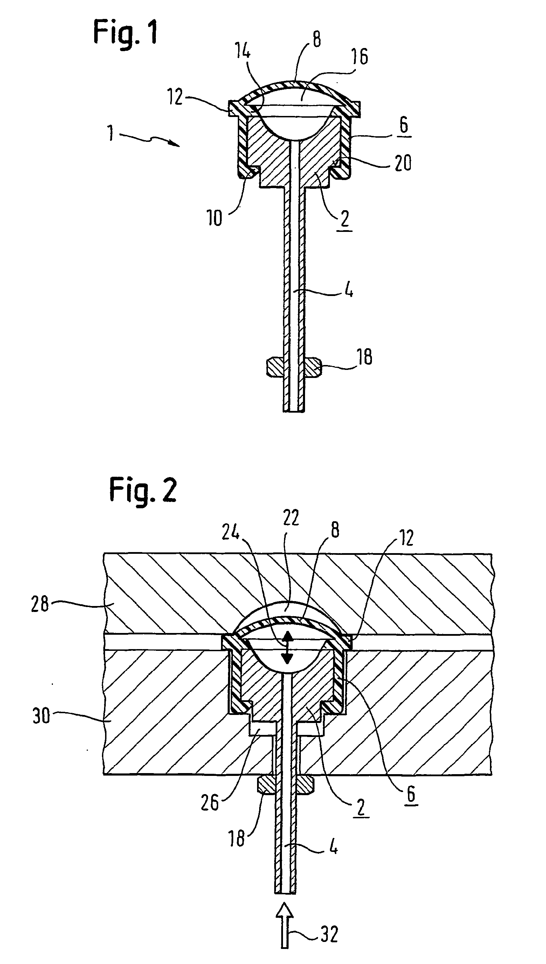

Figur 1 zeigt das Ventil 1 in Schnittansicht, die um eine senkrechte Achse rotationssymmetrisch ist. Das Ventil 1 besteht aus einem Ventilkörper 2 mit einem Druckkanal 4, der in einem Druckraum 16 endet. Über den Ventilkörper 2 stülpt sich eine Dichtkappe 6 mit einem verformbaren Bereich 8, der den Druckraum 16 begrenzt.

Der Druckkanal 4 des Ventilkörpers 2 ist langgestreckt ausgebildet, so daß er z.B. durch den Korpus oder eine Wandung eines geräteseitigen Gegenstückes einer Disposablekassette hindurchgesteckt werden kann und mit der Befestigungsmutter 18 festgeschraubt werden kann. Dazu ist auf der Außenwandung des Druckkanales 4 ein nicht eingezeichnetes Gewinde vorgesehen. Zur Abdichtung des Ventilkörpers 2 in dem geräteseitigen Gegenstück der Disposablekassette weist der Ventilkörper 2 Dichtflächen 20 auf. Die Dichtkappe 6 umfasst vorstehende Wulste 10, die den Ventilkörper 2 derart umfassen, daß sie an den Dichtflächen 20 anliegen und bei der Montage des Ventiles verpreßt werden.

Der in der Fig. 1 obere Bereich des Ventiles 1 ist der fluidkanalseitige Bereich. Zur Einpassung in den Fluidkanal ist ein Ansatz 12 der Dichtkappe 6 vorgesehen, wie es mit Bezug zur Fig. 2 erläutert werden wird. Am fluidkanalseitigen Ende des Ventilkörpers 2 liegt ein Vorsprung 14 der Dichtkappe 6 an.

In Fig. 2 ist ein erfindungsgemäßes Ventil schematisch im Einsatz gezeigt. 28 bezeichnet den Teil des Disposablenkassettenkorpus in schematischer Darstellung, in den die Flüssigkeitskanäle 22 eingeprägt sind. 30 bezeichnet das entsprechende geräteseitige Gegenstück des Disposablekassettenkorpus, das mit an sich bekannten, nicht gezeigten Befestigungsmitteln gegen das Teil 28 verpreßt ist. In eine entsprechend geformte Aufnahme 26 des Gegenstückes 30 ist das Ventil 1 eingeführt und mit der Befestigungsmutter 18 festgeschraubt. Der Ansatz 12 liegt an den Rändern des Flüssigkeitskanales 22 an. Die Bewegung des verformbaren Bereiches 8 bei Anlegen eines Überdruckes bzw. Unterdruckes oder bei Entlüftung des Druckkanales 4 ist mit dem Pfeil 24 bezeichnet. Mit 32 ist die Richtung bezeichnet, in der Druck angelegt wird, um das Ventil zu schließen. In Fig. 2 ist die Aufnahme 26 in dem Gegenstück 30 rotationssymmetrisch um den Druckkanal 4 des Ventils 1, während sich der Flüssigkeitskanal 22 bei dem gezeigten Beispiel senkrecht zur Figurenebene erstreckt.

Abweichend von der gezeigten Ausführungsform kann entweder in dem Diposablekassettenkorpus 28 oder in dem geräteseitigen Gegenstück 30 eine Aussparung zur Aufnahme des Ansatzes 12 vorgesehen sein. Ebenso ist es möglich, dass der Ansatz 12 in eine zwischen dem Disposablekorpus 28 und dem geräteseitigen Gegenstück 30 befindlichen Abdeckmatte in einer entsprechenden Öffnung aufgenommen wird.

In Figur 2 ist der Übersichtlichkeit halber eine an sich bekannte Disposablefolie nicht gezeigt, die den Fluidkanal 22 gegen die Umgebung abschließt. Eine solche Disposablefolie kann auf derjenigen Seite des Disposablekorpus 28 befestigt sein, die mit dem geräteseitigen Gegenstück 30 verpresst wird. Dabei muss die Disposablefolie derart ausreichend flexibel sein, dass sie der Verformung des Dichtbereiches 8 folgen kann.

Zum Betrieb des Ventiles 1 mit einer Disposablekassette wird der Ventilkörper 2 durch die Aufnahme 26 des geräteseitigen Gegenstückes 30 gesteckt, so daß sich der Druckkanal 4 durch das Gegenstück 30 erstreckt. Die Befestigungsmutter 18 wird angezogen, so daß die Dichtungswülste 10 zwischen dem Ventilkörper 2 und dem Gegenstück 30 dichten. Durch einfaches Anschrauben der Befestigungsmutter 18 ist also eine dichte und verläßliche Verbindung des Ventils 1 mit dem geräteseitigen Gegenstück 30 der Disposablekassette gegeben.

Das Gegenstück 30 mit dem Ventil 1 wird mit dem Disposablekassettenkorpus 28 verpreßt, wobei die Dichtkappe 6 mit den Ansätzen 12 mit den Rändern des Flüssigkeitskanales 22 anliegt. Durch die Verpressung des Gegenstückes 30 an den Disposablekassettenkorpus 28 werden in der Regel gleichzeitig an den gewünschten Stellen mehrere Ventile 1 in entsprechende Flüssigkeitskanäle 22 eingepaßt.

Durch den Fluidkanal 22 fließt im geöffneten Zustand des Ventiles 1 z.B. die Dialyseflüssigkeit. Wird nun in Pfeilrichtung 32 an den Druckkanal 4 Überdruck angelegt, so verformt sich der Dichtbereich 8 in den Flüssigkeitskanal 22 hinein, bis dieser schließlich verschlossen ist. Durch den Vorsprung 14 wird die Belastung der Kappe bei Verpressung auf dem Kanal minimiert, ohne daß die Bewegung des verformbaren Bereiches wesentlich beeinträchtigt würde. Ist an dem Disposablekorpus eine Disposablefolie vorgesehen, so verformt sich diese zusammen mit der Dichtkappe in den Flüssigkeitskanal hinein.

Soll der Fluidkanal 22 wieder geöffnet werden, so wird der Druckkanal 4 entlüftet und der verformbare Bereich 8 der Dichtkappe 6 entspannt sich. Durch Anlegen von Unterdruck an den Druckkanal 4 legt sich der verformbare Bereich 8 an die Auswölbung des Druckraumes 16 an und erhöht den Querschnitt des Fluidkanales 22 dementsprechend. Durch einfaches Anlegen und Entfernen einer Druckbeaufschlagung an den Druckkanal 4 läßt sich also der Durchfluß durch den Fluidkanal 22 steuern.

Bei entfernter Disposablekassette läßt sich das Ventil, z.B. zur Wartung oder bei einer Fehlfunktion durch einfaches Lösen der Befestigungsmutter 18 entfernen bzw. Auswechseln.

Die Dichtkappe ist ein einfaches kostengünstiges Formteil, das auf Grund seiner geschlossenen Ausführung leicht zu reinigen ist und damit den hygienischen Anforderungen bei der Dialyse genügt, das aber bei Bedarf auch leicht ausgewechselt werden kann.

Beim Wiederverschließen der Disposablekassette durch Befestigung des Gegenstückes 30 an dem Korpus 28 fügt sich das Ventil durch die Verpressung des Ansatzes 12 mit dem Rand des Fluidkanales 22 sehr gut ein. Aufgrund der elastischen Ausdehnung des verformbaren Bereiches 8 der Dichtkappe 6 besteht ein sehr guter Toleranzausgleich sowohl in der Tiefe des Kanales 22 als auch gegenüber seitlichem Versatz, ohne daß ein wesentlicher zusätzlicher Kraftaufwand entstehen würde. Der verformbare Bereich 8 gewährleistet, dass nur geringe Kräfte zum Absperren des Fluidkanales 22 nötig sind.

## Patentansprüche

1. Ventil zum Verschluß von Fluidkanälen, insbesondere zum Einsatz mit Disposablekassetten für extrakorporale Kreisläufe, das einen Ventilkörper (2) mit einem Druckkanal (4) und einer Dichtkappe (6) umfaßt und derart ausgestaltet ist, daß sich zwischen dem Druckkanal (4) und der Dichtkappe (6) ein Druckraum aufbauen kann, wobei die Dichtkappe (6) einen verformbaren Dichtbereich (8) aufweist, der derart verformbar ist, daß ein Fluidkanal (22) verschließbar ist,
**dadurch gekennzeichnet,**
**daß** die Dichtkappe (6) von vorne derart über den Ventilkörper (2) gestülpt ist, daß sie einerseits den Ventilkörper (2) umfaßt und Wulste (10) aufweist, die an rückwärtigen Dichtflächen (20) des Ventilkörpers anliegen, und andererseits über einen Ansatz (12) in den zu verschließenden Fluidkanal (22) einpaßbar ist.

2. Ventil nach Anspruch 1, bei dem die Dichtkappe (6) von dem Ventilkörper (2) lösbar ausgestaltet ist.

3. Ventil nach einem der Ansprüche 1 oder 2 mit einer Befestigungsvorrichtung (18) zum Befestigen des Ventilkörpers (2) in einer Aufnahme (26).

4. Ventil nach einem der Ansprüche 1 - 3, wobei die Dichtfläche (20) am Ventilkörper (2) derart von Wülsten (10) der Dichtkappe (6) umfaßt wird, daß bei Befestigung des Ventilkörpers (2) in einer Aufnahme (26) die Dichtkappe (6) an die Dichtfläche (20) gepreßt wird.

5. Ventil nach einem der Ansprüche 1 bis 3, wobei die Dichtkappe (6) einen Vorsprung (14) zur Verhinderung des direkten Kontaktes des Ventilkörpers (2) mit dem Dichtbereich (8) aufweist.

## Claims

1. A valve for the closure of fluid passages, in particular for use with disposable cassettes for extracorporeal circulations, which includes a valve body (2) with a pressure passage (4) and a sealing cap (6) and which is of such a configuration that a pressure chamber can be formed between the pressure passage (4) and the sealing cap (6), wherein the sealing cap (6) has a deformable sealing region (8) which is deformable in such a way that a fluid passage (22) is closable,
**characterised in that**
the sealing cap (6) is fitted over the valve body (2) from the front in such a way that on the one hand it embraces the valve body (2) and has ridges (10) which bear against rearward sealing surfaces (20) of the valve body and on the other hand it can be fitted by way of a shoulder (12) into the fluid passage (22) to be closed.

2. A valve according to claim 1 wherein the sealing cap (6) is adapted to be releasable from the valve body (2).

3. A valve according to one of claims 1 and 2 comprising a fixing device (18) for fixing the valve body (2) in a receiving means (26).

4. A valve according to one of claims 1 to 3 wherein the sealing surface (20) on the valve body (2) is embraced by ridges (10) on the sealing cap (6) in such a way that the sealing cap (6) is pressed against the sealing surface (20) when the valve body (2) is fixed in a receiving means (26).

5. A valve according to one of claims 1 to 3 wherein the sealing cap (6) has a projection (14) for preventing direct contact of the valve body (2) with the sealing region (8).

## Revendications

1. Soupape pour fermer des canaux de fluide, en particulier pour utilisation avec des cassettes jetables pour des circulations extra-corporelles, qui comprend un corps de soupape (2) avec un canal de pression (4) et un capuchon d'étanchéité (6) et qui est réalisée de façon qu'une enceinte de pression puisse se former entre le canal de pression (4) et le capuchon d'étanchéité (6), où le capuchon d'étanchéité (6) présente une zone d'étanchéité déformable (8), qui peut être déformée de telle sorte qu'un canal de fluide (22) peut être fermé,
**caractérisée en ce que** le capuchon d'étanchéité (6) est retourné depuis l'avant de telle sorte sur le corps de soupape (2) qu'il entoure, d'une part, le corps de soupape (2) et présente des bourrelets (10) qui s'appliquent aux faces d'étanchéité arrière (20) du corps de soupape, et qu'il peut être adapté, d'autre part, par un bout rapporté (12) dans le canal de fluide (22) à fermer.

2. Soupape selon la revendication 1, où le capuchon d'étanchéité (6) est réalisé pour qu'il puisse être détaché du corps de soupape (2).

3. Soupape selon l'une des revendications 1 ou 2, avec un dispositif de fixation (18) pour fixer le corps de soupape (2) dans un logement (26).

4. Soupape selon l'une des revendications 1 à 3, où la face d'étanchéité (20) au corps de soupape (2) est entourée de telle sorte par les bourrelets (10) du capuchon d'étanchéité (6) que lors de la fixation du corps de soupape (2) dans un logement (26), le capuchon d'étanchéité (6) est appliqué par pression à la face d'étanchéité (20).

5. Soupape selon l'une des revendications 1 à 3, où le capuchon d'étanchéité (6) présente une saillie (14) pour empêcher le contact direct du corps de soupape (2) avec la zone d'étanchéité (8).
